(19) Europäisches Patentamt / European Patent Office / Office européen des brevets

(11) **EP 1 517 697 B1**

(12) **EUROPÄISCHE PATENTSCHRIFT**

(45) Veröffentlichungstag und Bekanntmachung des Hinweises auf die Patenterteilung:
**10.10.2012 Patentblatt 2012/41**

(51) Int Cl.:
***A61K 31/7032*** *(2006.01)*    ***A61K 38/28*** *(2006.01)*

(21) Anmeldenummer: 03732536.2

(86) Internationale Anmeldenummer:
**PCT/EP2003/005887**

(22) Anmeldetag: **05.06.2003**

(87) Internationale Veröffentlichungsnummer:
**WO 2003/105888 (24.12.2003 Gazette 2003/52)**

(54) **SAURE INSULINZUBEREITUNGEN MIT VERBESSERTER STABILITÄT**

ACIDIC INSULIN PREPARATIONS WITH IMPROVED STABILITY

PREPARATIONS ACIDES D'INSULINE AYANT UNE MEILLEURE STABILITE

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **18.06.2002 DE 10227232**

(43) Veröffentlichungstag der Anmeldung:
**30.03.2005 Patentblatt 2005/13**

(60) Teilanmeldung:
**10178842.0 / 2 305 288**

(73) Patentinhaber: **Sanofi-Aventis Deutschland GmbH**
**65929 Frankfurt am Main (DE)**

(72) Erfinder:
• **BRUNNER-SCHWARZ, Anette**
**89077 ULM (DE)**
• **LILL, Norbert**
**61476 Kronberg (DE)**

(56) Entgegenhaltungen:
WO-A-92/00321    DE-A- 4 140 186
FR-A- 2 456 522    US-A- 4 994 439

EP 1 517 697 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine pharmazeutische Formulierung enthaltend ein Polypeptid ausgewählt aus einer Gruppe enthaltend Insulin, einen Insulinmetaboliten, ein Insulinanalogon, ein Insulinderivat oder Kombinationen davon; ein Tensid oder Kombinationen mehrerer Tenside; optional ein Konservierungsmittel oder Kombinationen mehrerer Konservierungsmittel; und optional ein Isotonisierungsmittel, Puffer oder weitere Hilfsstoffe oder Kombinationen davon, wobei die pharmazeutische Formulierung einen pH-Wert im sauren Bereich aufweist.

Diese Formulierungen können zur Behandlung von Diabetes eingesetzt werden, und sind besonders geeignet für Zubereitungen, bei denen eine hohe Stabilität gegenüber thermischer und/oder physikalisch-mechanischer Belastung erforderlich ist. Die Erfindung betrifft ebenfalls parenterale Zubereitungen, die solche Formulierungen enthalten und bei Diabetes angewandt werden können sowie Methoden, um die Zubereitungen herzustellen und die Stabilität von Insulinzubereitungen zu verbessern.

Weltweit leiden etwa 120 Mio. Menschen an Diabetes mellitus. Darunter sind etwa 12 Mio. Typ I-Diabetiker, für die die Substitution der fehlenden endokrinen Insulinsekretion die einzige derzeit mögliche Therapie darstellt. Die Betroffenen sind lebenslang, in der Regel mehrmals täglich, auf Insulininjektionen angewiesen. Im Gegensatz zum Typ I-Diabetes besteht beim Typ II-Diabetes nicht grundsätzlich ein Mangel an Insulin, jedoch wird in einer Vielzahl von Fällen, vor allem im fortgeschrittenen Stadium, die Behandlung mit Insulin, gegebenenfalls in Kombination mit einem oralen Antidiabetikum, als günstigste Therapieform angesehen.

Beim Gesunden ist die Insulinfreisetzung durch den Pankreas strikt an die Konzentration der Blutglucose gekoppelt. Erhöhte Blutglucosespiegel, wie sie nach Mahlzeiten auftreten, werden durch eine entsprechende Steigerung der Insulinsekretion rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten und die hepatische Glucoseproduktion in der Nacht niedrig zu halten. Der Ersatz der körpereigenen Insulinsekretion durch exogene, meist subcutane Applikation von Insulin erreicht in der Regel die oben beschriebene Qualität der physiologischen Regulation der Blutglucose nicht annähernd. Häufig kommt es zu Entgleisungen der Blutglucose nach oben oder unten, die in ihren schwersten Formen lebensbedrohlich sein können. Daneben stellen jedoch auch über Jahre erhöhte Blutglucosespiegel ohne anfängliche Symptome ein erhebliches Gesundheitsrisiko dar. Die großangelegte DCCT-Studie in den USA (The Diabetes Control and Complications Trial Research Group (1993) N. Engl. J. Med. 329, 977-986) wies eindeutig nach, daß chronisch erhöhte Blutglucosespiegel wesentlich für die Entwicklung diabetischer Spätschäden verantwortlich sind. Diabetische Spätschäden sind mikro- und makrovaskuläre Schädigungen, die sich u.U. als Retino-, Nephro-, oder Neuropathie manifestieren und zu Erblindung, Nierenversagen sowie dem Verlust von Extremitäten führen und darüber hinaus mit einem erhöhten Risiko für Herz/Kreislauferkrankungen einhergehen. Daraus ist abzuleiten, daß eine verbesserte Therapie des Diabetes in erster Linie darauf abzielen muß, die Blutglucose möglichst eng im physiologischen Bereich zu halten. Nach dem Konzept der intensivierten Insulintherapie soll dies durch mehrmals tägliche Injektionen von schnell und langsam wirkenden Insulinzubereitungen erreicht werden. Rasch wirkende Formulierungen werden zu den Mahlzeiten gegeben, um den postprandialen Anstieg der Blutglucose auszugleichen. Langsam wirkende Basalinsuline sollen die Grundversorgung mit Insulin insbesondere während der Nacht sicherstellen, ohne zu einer Hypoglykämie zu führen.

Insulin ist ein Polypeptid aus 51 Aminosäuren, die sich auf 2 Aminosäureketten verteilen: die A Kette mit 21 Aminosäuren und die B-Kette mit 30 Aminosäuren. Die Ketten sind durch 2 Disulfidbrücken miteinander verbunden. Insulinzubereitungen werden seit vielen Jahren zur Diabetestherapie eingesetzt. Dabei werden nicht nur natürlich vorkommende Insuline verwendet, sondern neuerdings auch Insulinderivate und -analoga.

Insulinanaloga sind Analoga von natürlich vorkommenden Insulinen, nämlich Humaninsulin oder tierischen Insulinen, welche sich durch Substitution wenigstens eines natürlich auftretenden Aminosäurerestes mit anderen Aminosäuren und/oder Addition/Entfernen wenigstens eines Aminosäurerestes von dem entsprechenden, ansonsten gleichen natürlich vorkommenden Insulin unterscheiden. Es kann sich dabei auch um Aminosäuren handeln, die nicht natürlich vorkommen.

Insulinderivate sind Derivate von natürlich vorkommendem Insulin oder einem Insulinanalogon, welche durch chemische Modifizierung erhalten werden. Die chemische Modifikation kann z.B. in der Addition einer oder mehrerer bestimmter chemischer Gruppen an eine oder mehrere Aminosäuren bestehen. In der Regel haben Insulinderivate und Insulinanaloga gegenüber humanem Insulin eine etwas veränderte Wirkung.

Insulinanaloga mit beschleunigtem Wirkungseintritt werden in EP 0 214 826, EP 0 375 437 und EP 0 678 522 beschrieben. EP 0 124 826 bezieht sich u.a. auf Substitutionen von B27 und B28. EP 0 678 522 beschreibt Insulinanaloga, die in der Position B29 verschiedene Aminosäuren, vorzugsweise Prolin, aufweisen, jedoch nicht Glutaminsäure.

EP 0 375 437 umfaßt Insulinanaloga mit Lysin oder Arginin in B28, die optional zusätzlich in B3 und/oder A21 modifiziert sein können.

In der EP 0 419 504 werden Insulinanaloga offenbart, die gegen chemische Modifikationen geschützt sind, in dem Asparagin in B3 und wenigstens eine weitere Aminosäure in den Positionen A5, A15, A18 oder A21 verändert sind.

In der Regel haben Insulinderivate und Insulinanaloga gegenüber humanem Insulin eine etwas veränderte Wirkung.

In der WO 92/00321 werden Insulinanaloga beschrieben, bei denen wenigstens eine Aminosäure der Positionen B1-B6 durch Lysin oder Arginin ersetzt ist. Derartige Insuline weisen gemäß WO 92/00321 eine verlängerte Wirkung auf. Eine verzögerte Wirkung weisen auch die in der EP-A 0 368 187 beschriebenen Insulinanaloga auf. Die auf dem Markt befindlichen Insulinzubereitungen von natürlich vorkommenden Insulinen zur Insulinsubstitution unterscheiden sich in der Herkunft des Insulins (z.B. Rind, Schwein, Humaninsulin), sowie der Zusammensetzung, womit das Wirkprofil (Wirkeintritt und Wirkdauer) beeinflusst werden kann. Durch Kombination verschiedener Insulinpräparate lassen sich unterschiedlichste Wirkprofile erzielen und möglichst physiologische Blutzuckerwerte einstellen. Die rekombinante DNA Technologie ermöglicht heutzutage die Herstellung von solchen modifizierten Insulinen. Hierzu zählt Insulin Glargin (Gly (A21)-Arg(B31)-Arg(B32)-Humaninsulin) mit einer verlängerten Wirkdauer. Insulin Glargin wird als saure, klare Lösung injiziert und fällt aufgrund seiner Lösungseigenschaften im physiologischen pH-Bereich des Subkutangewebes als stabiles Hexamerassoziat aus. Insulin Glargin wird einmal täglich injiziert und zeichnet sich gegenüber anderen langwirksamen Insulinen durch sein flaches Serumprofil und die damit verbundene Reduktion der Gefahr nächtlicher Hypoglykämien aus (Schubert-Zsilavecz et al., 2:125-130(2001)).

Die spezifische Zubereitung des Insulin Glargins, die zur verlängerten Wirkdauer führt, ist im Gegensatz zu bisher beschriebenen Zubereitungen durch einen klare Lösung mit saurem pH-Wert gekennzeichnet. Gerade bei saurem pH-Wert zeigen Insuline jedoch eine verringerte Stabilität und eine erhöhte Aggregationsneigung bei thermischer und physikalisch-mechanischer Belastung, die sich in Form von Trübungen und Ausfällungen (Partikelbildungen) bemerkbar machen kann (Brange et al., J. Ph.Sci 86:517-525(1997)).

Die Aggregationsneigung kann zusätzlich durch hydrophobe Oberflächen, die in Kontakt mit der Lösung stehen, gefördert werden (Sluzky et al., Proc.Natl.Acad.Sci. 88:9377-9381 (1991). Als hydrophobe Oberflächen können die Glasgefäße der Zubereitungen, das Stopfenmaterial der Verschußkappen oder die Grenzfläche der Lösung zum Luftüberstand betrachtet werden. Daneben können feinste Silkonöltröpfchen als zusätzliche hydrophobe Aggregationskeime bei der Entnahme der täglichen Insulindosis über handelsübliche, silkonisierte Insulinspritzen fungieren und den Prozeß beschleunigen.

WO 01/43762 beschreibt wässrige, parenterale pharmazeutische Zubereitungen enthaltend ein Polypeptid und Glycerin, bei denen die Stabilisierung der Zubereitung durch Abreinigung destabilisierender Bestandteile des Glycerins erreicht werden soll. WO 00/23098 beschreibt Insulinzubereitungen stabilisiert mit Polysorbat 20 oder Poloxamer 188 für pulmonale Applikation, beschreibt jedoch nicht die Stabilisierung in saurer Lösung gegen Aggregationskeime.

Die internationale Patentanmeldung PCT/EP02/02625 (unveröffentlicht) beschreibt zinkfreie und zinkarme Insulinzubereitungen mit durch den Zusatz von Tensiden verbesserter Stabilität bei Raum- und Körpertemperatur und mechanischer Belastung, beschreibt jedoch nicht die Stabilisierung saurer Insulinzubereitungen gegenüber hydrophoben Aggregationskeimen.

Der vorliegenden Erfindung lag somit die Aufgabe zugrunde, Zubereitungen für im Sauren lösliche Insuline mit Tensiden zu finden, die sich durch eine hohe Langzeitstabilität gegenüber Belastungen durch Temperatur oder physikalisch-mechanischer Stressung auszeichnen und eine hohe Belastung mit hydrophoben Aggregationskeimen tolerieren.

Es wurde nun überraschenderweise gefunden, dass der Zusatz von Tensiden die Stabilität von sauren Insulinzubereitungen stark erhöhen kann und damit sogar Zubereitungen hergestellt werden können, die über mehrere Monate bei Temperaturbelastung die überlegene Stabilität gegenüber hydrophoben Aggregationskeimen garantieren.

Die pharmazeutischen Zubereitungen enthalten 60-6000 nmol/ml, bevorzugt 240-3000 nmol/ml eines Insulins, eines Insulinmetaboliten, eines Insulinanalogons oder eines Insulinderivats.

Als Tenside können Tween® 20 und Tween® 80 Verwendung finden. Die Tenside liegen in der pharmazeutischen Zusammensetzung in einer Konzentration von 5 - 200 μg/ml, bevorzugt von 5 -120μg/ml und besonders bevorzugt von 20 - 75 μg/ml vor.

Die Zubereitung kann desweiteren Konservierungsmittel (z.B. Phenol, Kresol, Parabene), Isotonisierungsmittel (z.B. Mannitol, Sorbitol, Lactose, Dextrose, Trehalose, Natriumchlorid, Glycerol). Puffersubstanzen, Salze, Säuren und Laugen sowie weitere Hilfstoffe enthalten. Diese Substanzen können jeweils einzeln oder auch als Mischungen vorliegen.

Glycerol, Dextrose, Lactose, Sorbitol und Mannitol liegen üblicherweise in der pharmazeutischen Zubereitung in einer Konzentration von 100 - 250 mM, NaCl in einer Konzentration bis zu 150 mM vor. Puffersubstanzen, wie z.B. Phosphat-, Acetat-, Citrat, Arginin , Glycylglycin oder TRIS (d.h. 2-Amino-2-Hydroxymethyl-1,3,-Propandiol) Puffer sowie entsprechende Salze, sind in einer Konzentration von 5 - 250 mM, bevorzugt 10 - 100 mM vorhanden. Weitere Hilfstoffe können unter anderem sein Salze oder Arginin.

In dem US-Patent 4,994,439 werden Zusammensetzungen von Proteinen oder Peptiden für die Verabreichung durch Membranen offenbart, die niedrige Toxizität und eine effiziente Permeation zeigen, wobei eine Mischung eines Gallensäuresalzes oder -fusidats mit einem nicht ionischen Detergenz enthalten ist.

In der französischen Patentanmeldung mit der Offenlegungssnummer 2 456 522 werden Insulinpräparate mit Hilfsstoffen für die Schleimhautresorption offenbart. Diese Hilfsstoffe stellen anorganische Säuren und verschiedene Carbonsäuren dar.

In der deutschen Patentanmeldung mit der Offenlegungsnummer 41 40 186 A1 werden perorale Applikationsformen mit einer Matrix aus Gelatine oder einem Gelatinederivat offenbart, bei denen der Wirkstoff, insbesondere Insulin, in der Matrix verteilt ist.

Gegenstand der Erfindung ist daher eine pharmazeutische Formulierung enthaltend

# Gly(A21), Arg(B31), Arg(B32)-Humaninsulin

ein Tensid, ausgewählt aus einer Gruppe enthaltend Tween® 20 und Tween® 80 in einer Konzentration von 5 - 200 μg/ml; optional ein Konservierungsmittel oder Kombinationen mehrerer Konservierungsmittel; und optional ein Isotonisierungsmittel, Puffer oder weitere Hilfsstoffe oder Kombinationen davon, wobei die pharmazeutische Formulierung eine klare Lösung ist, die einen pH-Wert im sauren Bereich (pH 3,5-4,5) aufweist.

Bevorzugt ist eine solche pharmazeutische Formulierung, bei der das Konservierungsmittel ausgewählt wird aus einer Gruppe enthaltend Phenol, Kresol, Parabene; wobei das Isotonisierungsmittel ausgewählt wird aus einer Gruppe enthaltend Mannitol, Sorbitol, Natriumchlorid, Glycerol; wobei die Hilfsstoffe ausgewählt werden aus einer Gruppe enthaltend Puffersubstanzen, Säuren, Laugen.

Ein weiterer Gegenstand der Erfindung ist eine pharmazeutische Formulierung wie oben beschrieben, bei der das Insulin, das Insulinanalogon, der aktive Insulinmetabolit und/oder das Insulinderivat in einer Konzentration von 60 - 6000 nmol/ml, vorzugsweise in einer Konzentration von 240 - 3000 nmol/ml vorliegt (dies entspricht etwa einer Konzentration von 1,4 - 35 mg/ml oder 40 - 500 Einheiten/ml); bei der das Tensid in einer Konzentration von 5 - 200 μg/ml, bevorzugt von 5 - 120μg/ml und besonders bevorzugt von 20 - 75 μg/ml vorliegt.

Ein weiterer Gegenstand der Erfindung ist eine pharmazeutische Formulierung wie oben ausgeführt, bei der Glycerol und/oder Mannitol in einer Konzentration von 100 - 250 mM, und/oder NaCl vorzugsweise in einer Konzentration bis zu 150 mM vorliegt.

Ein weiterer Gegenstand der Erfindung ist eine pharmazeutische Formulierung wie oben ausgeführt, bei der eine Puffersubstanz in einer Konzentration von 5 - 250 mM vorliegt.

Ein weiterer Gegenstand der Erfindung ist eine pharmazeutische Insulinformulierung, die weitere Zusätze wie z.B. Salze enthält, welche die Freigabe von Insulin retardieren.

Auch Mischungen aus solchen Retardinsulinen mit oben beschriebenen Formulierungen sind darin eingeschlossen.

Ein weiterer Gegenstand der Erfindung ist eine Methode zur Herstellung solcher pharmazeutischer Formulierungen. Letztere sind zur Behandlung von Diabetes meilitus geeignet. Ein weiterer Gegenstand der Erfindung ist die Verwendung bzw. der Zusatz von Tensiden als Stabilisator während des Herstellungsprozesses von Insulin, Insulinanaloga oder Insulinderivaten oder deren Zubereitungen.

Im folgenden wird die Anmeldung anhand einiger Beispiele beschrieben, die keinesfalls beschränkend wirken sollen.

Beispiele:

Die folgenden Beispiele sollen den Erfindungsgedanken näher erläutern, ohne beschränkend zu wirken.

Vergleichsuntersuchungen: Es werden verschiedene Zubereitungen mit dem Insulinanalogon Insulin Glargin (Gly(A21),Arg(B31),Arg(B32)-Humaninsulin) hergestellt. Dazu wird Insulin Glargin in einem Teil Wasser für Injektionszwecke suspendiert, bei pH 3 -4 gelöst, die anderen Bestandteile werden zugefügt, der pH Wert mit Salzsäure/NaOH auf 4,0 +/- 0,2 eingestellt und auf das Endvolumen aufgefüllt. Die Konzentration von Insulin glargin beträgt bei jedem der unten beschriebenen Versuche 3,6378 mg/ml (entspricht 100 Einheiten/ml). Eine zweite Zubereitung wird identisch hergestellt, jedoch wird noch eine bestimmte Menge eines Tensids hinzugegeben. Die Lösungen werden in 10 ml Glasgefäße (Vials) abgefüllt und verbördelt. Diese Gefässe werden nun simulierten in use- bzw. physikalischmechanischen Stressbedingungen ausgesetzt:

1. In use-Test: Die Gefäße werden in Stülpdeckelschachteln einsortiert und während des Untersuchungszeitraumes von 28 Tagen bei + 25° C und kontrollierter Raumfeuchte unter Ausschluß von Licht gelagert. Zur Simulation der Patientenentnahme werden täglich einmal ca. 5 IU der Lösung mit einer handelsüblichen Insulinspritze entnommen und verworfen. Am Anfang und Ende der Arbeitswoche wird dieses Vorgehen zweimal ausgeführt, um die Entnahme am Wochenende nachzustellen. Vor jeder Entnahme erfolgt die visuelle Beurteilung der Lösung in den Gefäßen auf Trübung und/oder Partikelbildung.

2. Schütteltest: Die Gefäße werden in einer Stülpdeckeischachtel liegend auf einen Laborschüttler mit Inkubator und Thermostat gestellt und bei 25°C mit 90 Bewegungen/min parallel zur Horizontalbewegung über einen Zeitraum von 10 Tagen geschüttelt. Nach definierten Zeiten wird der Trübungswert der Proben mittels eines Labor-Trübungsphotometers (Nephelometer) in Formaldazin Nephelometrischen Einheiten (Formaldazine Nephelometric Unit = FNU) bestimmt. Der Trübungswert entspricht der Intensität der gestreuten Strahlung des eingestrahlten Lichtes an suspendierten Teilchen in der Probe.

Beispiel 1: Stabilisierung der In use-Periode von Insulin glargin mit Polysorbat 20 (Tween® 20)

a) Die Lösung wird über eine Kombination aus 0,2 $\mu$m und 0,1 $\mu$m Filter steril filtriert. Anschliessend wird sie in 10 ml Injektionsfläschchen gefüllt und mit Bördelkappen mit eingelegter Dichtscheibe verschlossen.
b) Eine Vergleichslösung wird identisch hergestellt, jedoch wird in Wasser für Injektionszwecke zuerst eine geeignete Menge Tensid (10 - 30 ppm Polysorbat 20) suspendiert
Die Muster werden bei +5°C , 25°C und 37°C für einen festgelegten Zeitraum eingelagert. Jeweils 10 Muster werden anschliessend einem in use-Test unterzogen. Die Ergebnisse sind in nachfolgenden Tabelle wiedergegeben.

*Lagerung 3 Monate bei 5°C*

| Prüfmuster | Anzahl Vials mit Partikelbildung nach | | | |
|---|---|---|---|---|
| | 7 Tagen | 14 Tagen | 21 Tagen | 28 Tagen |
| Insulin Glargin | 7 | 10 | 10 | 10 |
| Insulin Glargin + 0,010 mg/ml Polysorbat 20 | 0 | 0 | 0 | 0 |
| Insulin Glargin + 0,015 mg/ml Polysorbat 20 | 0 | 0 | 0 | 0 |
| Insulin Glargin + 0,020 mg/ml Polysorbat 20 | 0 | 0 | 0 | 1 |
| Insulin Glargin + 0,030 mg/ml Polysorbat 20 | 0 | 0 | 0 | 0 |

*Lagerung 6 Monate bei 5°C*

| Prüfmuster | Anzahl Vials mit Partikelbildung nach | | | |
|---|---|---|---|---|
| | 7 Tagen | 14 Tagen | 21 Tagen | 28 Tagen |
| Insulin Glargin | 1 | 10 | 10 | 10 |
| Insulin Glargin + 0,010 mg/ml Polysorbat 20 | 0 | 0 | 0 | 1 |
| Insulin Glargin + 0,015 mg/ml Polysorbat 20 | 0 | 0 | 0 | 0 |
| Insulin Glargin + 0,020 mg/ml Polysorbat 20 | 0 | 0 | 0 | 1 |
| Insulin Glargin + 0,030 mg/ml Polysorbat 20 | 0 | 0 | 1 | 0 |

Lagerung 3 Monate bei 25°C

| Prüfmuster | Anzahl Vials mit Partikelbildung nach | | | |
|---|---|---|---|---|
| | 7 Tagen | 14 Tagen | 21 Tagen | 28 Tagen |
| Insulin Glargin | 9 | 10 | 10 | 10 |

(fortgesetzt)

| Prüfmuster | Anzahl Vials mit Partikelbildung nach | | | |
|---|---|---|---|---|
| | 7 Tagen | 14 Tagen | 21 Tagen | 28 Tagen |
| Insulin Glargin + 0,010 mg/ml Polysorbat 20 | 2 | 2 | 2 | 2 |
| Insulin Glargin + 0,015 mg/ml Polysorbat 20 | 0 | 0 | 0 | 1 |
| Insulin Glargin + 0,020 mg/ml Polysorbat 20 | 0 | 0 | 0 | 0 |
| Insulin Glargin + 0,030 mg/ml Polysorbat 20 | 0 | 0 | 0 | 0 |

Lagerung 6 Monate bei 25°C

| Prüfmuster | Anzahl Vials mit Partikelbildung nach | | | |
|---|---|---|---|---|
| | 7 Tagen | 14 Tagen | 21 Tagen | 28 Tagen |
| Insulin Glargin | 10 | 10 | 10 | 10 |
| Insulin Glargin + 0,010 mg/ml Polysorbat 20 | 0 | 0 | 0 | 1 |
| Insulin Glargin + 0,015 mg/ml Polysorbat 20 | 0 | 0 | 1 | 0 |
| Insulin Glargin + 0,020 mg/ml Polysorbat 20 | 0 | 0 | 0 | 0 |
| Insulin Glargin + 0,030 mg/ml Polysorbat 20 | 0 | 0 | 0 | 0 |

Lagerung 1 Monat bei 37°C

| Prüfmuster | Anzahl Vials mit Partikelbildung nach | | | |
|---|---|---|---|---|
| | 7 Tagen | 14 Tagen | 21 Tagen | 28 Tagen |
| Insulin Glargin | 0 | 10 | 10 | 10 |
| Insulin Glargin + 0,010 mg/ml Polysorbat 20 | 0 | 3 | 3 | 5 |
| Insulin Glargin + 0,015 mg/ml Polysorbat 20 | 0 | 0 | 0 | 0 |
| Insulin Glargin + 0,020 mg/ml Polysorbat 20 | 0 | 0 | 0 | 0 |
| Insulin Glargin + 0,030 mg/ml Polysorbat 20 | 0 | 0 | 0 | 0 |

Lagerung 3 Monate bei 37°C

| Prüfmuster | Anzahl Vials mit Partikelbildung nach | | | |
|---|---|---|---|---|
| | 7 Tagen | 14 Tagen | 21 Tagen | 28 Tagen |
| Insulin Glargin | 5 | 9 | 10 | 10 |
| Insulin Glargin + 0,010 mg/ml Polysorbat 20 | 1 | 1 | 1 | 1 |
| Insulin Glargin + 0,015 mg/ml Polysorbat 20 | 0 | 0 | 0 | 0 |
| Insulin Glargin + 0,020 mg/ml Polysorbat 20 | 0 | 0 | 0 | 0 |
| Insulin Glargin + 0,030 mg/ml Polysorbat 20 | 0 | 0 | 0 | 0 |

Lagerung 6 Monate bei 37°C

| | Anzahl Vials mit Partikelbildung nach | | | |
|---|---|---|---|---|
| Prüfmuster | 7 Tagen | 14 Tagen | 21 Tagen | 28 Tagen |
| Insulin Glargin | 10 | 10 | 10 | 10 |
| Insulin Glargin + 0,010 mg/ml Polysorbat 20 | 0 | 0 | 0 | 0 |
| Insulin Glargin + 0,015 mg/ml Polysorbat 20 | 0 | 0 | 1 | 0 |
| Insulin Glargin + 0,020 mg/ml Polysorbat 20 | 0 | 0 | 0 | 0 |
| Insulin Glargin + 0,030 mg/ml Polysorbat 20 | 1 | 1 | 1 | 1 |

Ohne Zusatz von Polysorbat 20 kann in der Lösung bereits nach 7 Tagen in use Partikelbildung auftreten. Durch Zusatz von Polysorbat 20 kann die Partikelbildung während der in use-Periode deutlich zurückgedrängt werden.
Die stabilisierende Wirkung des Polysorbat 20 bleibt auch bei Einlagerung bei erhöhten Temperaturen für einen Zeitraum von 3 Monaten bestehen. Ein Nachlassen der stabilisierenden Wirkung durch mögliche Hydrolyse des Polysorbates im sauren Milieu der Lösung kann im Vergleich mit den Daten nach 1 Monat Einlagerung nicht festgestellt werden.

Beispiel 2: Stabilisierung von Insulin Glargin mit Polysorbat 20 bei physikalisch-mechanischer Stressbelastung

a) Die Lösung wird über eine Kombination aus 0,2 $\mu$m und 0,1 $\mu$m Filter steril filtriert. Anschliessend wird sie in 10 ml Injektionsfläschchen gefüllt und mit Bördelkappen mit eingelegter Dichtscheibe verschlossen.
b) Eine Vergleichslösung wird identisch hergestellt, jedoch wird in Wasser für Injektionszwecke zuerst eine geeignete Menge Tensid (0,010 - 0,030 mg/ml Polysorbat 20) suspendiert
Die Muster werden bei +5°C, 25°C und 37°C für einen festgelegten Zeitraum eingelagert. Jeweils 5 Muster werden anschliessend einem Schütteltest unterzogen. Die Ergebnisse sind in nachfolgenden Tabelle wiedergegeben, die Grenze 15 FNU entspricht Trübungen, die im Tageslicht erkennbar sind.

Lagerung 1 Monat bei 5°C

| | Anzahl Vials > 15 FNU | | | | | | | | |
|---|---|---|---|---|---|---|---|---|---|
| Prüfmuster | 0 Tage | 0,5 Tage | 1 Tag | 2 Tage | 3 Tage | 4 Tage | 6 Tage | 8 Tage | 10 Tage |
| Insulin Glargin | 0 | 0 | 0 | 2 | 3 | 3 | 4 | 4 | 4 |
| Insulin Glargin + 0,010 mg/ml Polysorbat 20 | 0 | 0 | 0 | 0 | 0 | 1 | 3 | 4 | 5 |
| Insulin Glargin + 0,015 mg/ml Polysorbat 20 | 0 | 0 | 0 | 0 | 0 | 0 | 0 | 0 | 0 |
| Insulin Glargin + 0,020 mg/ml Polysorbat 20 | 0 | 0 | 0 | 0 | 0 | 0 | 0 | 0 | 0 |
| Insulin Glargin + 0,030 mg/ml Polysorbat 20 | 0 | 0 | 0 | 0 | 0 | 0 | 0 | 0 | 0 |

Lagerung 1 Monat bei 25°C

| | Anzahl Vials > 15 FNU | | | | | | | | |
|---|---|---|---|---|---|---|---|---|---|
| Prüfmuster | 0 Tage | 0,5 Tage | 1 Tag | 2 Tage | 3 Tage | 4 Tage | 6 Tage | 8 Tage | 10 Tage |
| Insulin Glargin | 0 | 0 | 0 | 1 | 1 | 1 | 1 | 2 | 3 |
| Insulin Glargin + 0,010 mg/ml Polysorbat 20 | 0 | 0 | 0 | 0 | 0 | 0 | 1 | 2 | 3 |
| Insulin Glargin + 0,015 mg/ml Polysorbat 20 | 0 | 0 | 0 | 0 | 0 | 0 | 0 | 0 | 0 |
| Insulin Glargin + 0,020 mg/ml Polysorbat 20 | 0 | 0 | 0 | 0 | 0 | 0 | 0 | 0 | 0 |
| Insulin Glargin + 0,030 mg/ml Polysorbat 20 | 0 | 0 | 0 | 0 | 0 | 0 | 0 | 0 | 0 |

Lagerung 1 Monat bei 37°C

| | Anzahl Vials > 15 FNU | | | | | | | | |
|---|---|---|---|---|---|---|---|---|---|
| Prüfmuster | 0 Tage | 0,5 Tage | 1 Tag | 2 Tage | 3 Tage | 4 Tage | 6 Tage | 8 Tage | 10 Tage |
| Insulin Glargin | 0 | 0 | 0 | 2 | 5 | 5 | 5 | 5 | 5 |
| Insulin Glargin + 0,010 mg/ml Polysorbat 20 | 0 | 0 | 0 | 0 | 0 | 0 | 0 | 0 | 0 |
| Insulin Glargin + 0,015 mg/ml Polysorbat 20 | 0 | 0 | 0 | 0 | 0 | 0 | 0 | 0 | 0 |
| Insulin Glargin + 0,020 mg/ml Polysorbat 20 | 0 | 0 | 0 | 0 | 0 | 0 | 0 | 0 | 0 |
| Insulin Glargin + 0,030 mg/ml Polysorbat 20 | 0 | 0 | 0 | 0 | 0 | 0 | 0 | 0 | 0 |

Ohne Zusatz von Polysorbat 20 kann in der Lösung bereits nach 2 Tagen starker physikalisch-mechanischer Beanspruchung eine sichtbare Trübung auftreten. Durch Zusatz von Polysorbat 20 kann die Trübungsbildung während physikalisch-mechanischer Stressung deutlich verzögert werden.
Die stabilisierende Wirkung des Polysorbat 20 bleibt auch bei Einlagerung bei erhöhten Temperaturen bestehen.
Ein Nachlassen der stabilisierenden Wirkung durch mögliche Hydrolyse des Polysorbates im sauren Milieu der Lösung kann nicht festgestellt werden.

Beispiel 3: Vergleich der Stabilisierung der In use-Periode von Insulin glargin mit Polysorbat 20 (Tween® 20) und mit Polysorbat 80 (Tween® 20)

Jeweils 10 Vials zu 5 ml Insulin glargin-Injektionslösung öffnen und

a) Zugabe von 0,001 mg/ml Polysorbat 20
b) Zugabe von 0,01 mg/ml Polysorbat 20
c) Zugabe von 0,001 mg/ml Polysorbat 80
d) Zugabe von 0,01 mg/ml Polysorbat 80

in Form einer konzentrierten Stammlösung.

Die Muster werden anschliessend einem in use-Test unterzogen.

Die Ergebnisse sind in nachfolgenden Tabelle wiedergegeben.

| Prüfmuster | Vials mit Partikelbildung nach | | | |
|---|---|---|---|---|
| | 7 Tagen | 14 Tagen | 21 Tagen | 28 Tagen |
| Insulin Glargin + 0,001 mg/ml Polysorbat 20 | nein | ja | Ja, Partikel treten verstärkt auf | Ja, Partikel treten verstärkt auf |
| Insulin Glargin + 0,010 mg/ml Polysorbat 20 | nein | nein | nein | nein |
| Insulin Glargin + 0,001 mg/ml Polysorbat 80 | nein | ja | Ja, Partikel treten verstärkt auf | Ja, Partikel treten verstärkt auf |
| Insulin Glargin + 0,010 mg/ml Polysorbat 80 | nein | nein | nein | nein |

Ein Zusatz von Polysorbat 20 oder von Polysorbat 80 in einer Konzentration von 0,01 mg/ml sind gleichermaßen in der Lage die Lösung gegen Partikelbildung während der in use-Periode zu stabilisieren.

**Patentansprüche**

1.  Pharmazeutische Formulierung enthaltend Gly(A21), Arg(B31), Arg(B32)-Humaninsulin;
    ein Tensid, ausgewählt aus einer Gruppe enthaltend Tween® 20 und
    Tween® 80 in einer Konzentration von 5 - 200 μg/ml;
    optional ein Konservierungsmittel oder Kombinationen mehrerer Konservierungsmittel; und optional ein Isotonisierungsmittel, Puffer oder weitere Hilfsstoffe oder Kombinationen davon,
    wobei die pharmazeutische Formulierung eine klare Lösung ist, die einen pH-Wert im sauren Bereich (pH 3,5 - 4,5) aufweist.

2.  Pharmazeutische Formulierung gemäß Anspruch 1, wobei das Konservierungsmittel ausgewählt wird aus einer Gruppe enthaltend Phenol, Kresol, Chlorkresol, Benzylalkohol, Parabene.

3.  Pharmazeutische Formulierung gemäß einem der Ansprüche 1 oder 2, wobei das Isotonisierungsmittel ausgewählt wird aus einer Gruppe enthaltend Mannitol, Sorbitol, Lactose, Dextrose, Trehalose, Natriumchlorid, Glycerol.

4.  Pharmazeutische Formulierung gemäß einem der Ansprüche 1 bis 3, wobei die Hilfsstoffe ausgewählt werden aus einer Gruppe enthaltend Puffersubstanzen wie z.B. TRIS, Phosphat, Citrat, Acetat, Glycylglycin oder weiterer Stoffe wie Säuren, Laugen, Salze.

5.  Pharmazeutische Formulierung gemäß einem der vorigen Ansprüche, bei der das Gly(A21), Arg(B31), Arg(B32)-Humaninsulin in einer Konzentration von 60 - 6000 nmol/ml vorliegt.

6.  Pharmazeutische Formulierung gemäß Anspruch 5, wobei das Gly(A21), Arg(B31), Arg(B32)-Humaninsulin in einer Konzentration von 240 - 3000 nmol/ml vorliegt.

7.  Pharmazeutische Formulierung gemäß Anspruch 6, bei der das Tensid in einer Konzentration von 5 -120 μg/ml vorliegt.

8.  Pharmazeutische Formulierung gemäß Anspruch 7, bei der das Tensid in einer Konzentration von 20 - 75 μg/ml vorliegt.

9. Pharmazeutische Formulierung gemäß einem oder mehreren der Ansprüche 4 - 8, bei der Glycerol und/oder Mannitol in einer Konzentration von 100 - 250 mM vorliegt.

10. Pharmazeutische Formulierung gemäß Anspruch 9, bei der NaCl in einer Konzentration von bis zu 150 mM vorliegt

11. Pharmazeutische Formulierung gemäß einem der vorigen Ansprüche, bei der eine Puffersubstanz in einer Konzentration von 5 - 250 mM vorliegt.

12. Pharmazeutische Formulierung gemäß einem der vorigen Ansprüche, bei der das Tensid Tween® 20 ist.

13. Pharmazeutische Formulierung gemäß einem der vorigen Ansprüche, bei der das Tensid Tween® 80 ist.

**Claims**

1. A pharmaceutical formulation comprising Gly(A21), Arg(B31), Arg(B32)-human insulin;
a surfactant selected from a group comprising Tween® 20 und Tween® 80 in a concentration of 5 - 200 $\mu$g/ml;
optionally a preservative or combinations of two or more preservatives; and optionally an isotonicizing agent, buffers or further excipients or combinations thereof,
the pharmaceutical formulation being a clear solution which has a pH in the acidic range (pH 3.5 - 4.5).

2. The pharmaceutical formulation as claimed in claim 1, the preservative being selected from a group comprising phenol, cresol, chlorocresol, benzyl alcohol, parabens.

3. The pharmaceutical formulation as claimed in either of claims 1 and 2, the isotonicizing agent being selected from a group comprising mannitol, sorbitol, lactose, dextrose, trehalose, sodium chloride, glycerol.

4. The pharmaceutical formulation as claimed in one of claims 1 to 3, the excipients being selected from a group comprising buffer substances such as, for example, TRIS, phosphate, citrate, acetate, glycylglycine or further substances such as acids, alkalis, salts.

5. The pharmaceutical formulation as claimed in one of the preceding claims, in which the Gly(A21), Arg(B31), Arg (B32)-human insulin is present in a concentration of 60 - 6000 nmol/ml.

6. The pharmaceutical formulation as claimed in claim 5, the Gly(A21), Arg(B31), Arg(B32)-human insulin being present in a concentration of 240 - 3000 nmol/ml.

7. The pharmaceutical formulation as claimed in claim 6, in which the surfactant is present in a concentration of 5 -120 $\mu$g/ml.

8. The pharmaceutical formulation as claimed in claim 7, in which the surfactant is present in a concentration of 20 - 75 $\mu$g/ml.

9. The pharmaceutical formulation as claimed in one or more of claims 4 - 8, in which glycerol and/or mannitol is present in a concentration of 100 - 250 mM.

10. The pharmaceutical formulation as claimed in claim 9, in which NaCl is present in a concentration of up to 150 mM.

11. The pharmaceutical formulation as claimed in one of the preceding claims, in which a buffer substance is present in a concentration of 5 - 250 mM.

12. The pharmaceutical formulation as claimed in one of the preceding claims, in which the surfactant is Tween® 20.

13. The pharmaceutical formulation as claimed in one of the preceding claims, in which the surfactant is Tween® 80.

**Revendications**

1. Composition pharmaceutique contenant de la Gly(A21), Arg(B31), Arg(B32)-insuline humaine ;
un tensioactif, choisi dans un groupe contenant le Tween® 20 et le Tween® 80, à une concentration de 5 - 200 μg/ml ;
en option un conservateur ou des associations de plusieurs conservateurs ; et en option un agent d'isotonie, un tampon ou d'autres adjuvants ou des associations de ceux-ci,
la composition pharmaceutique étant une solution limpide qui présente un pH dans l'intervalle acide (pH 3,5 - 4,5).

2. Composition pharmaceutique selon la revendication 1, dans laquelle le conservateur est choisi dans un groupe contenant le phénol, le crésol, le chlorocrésol, l'alcool benzylique, les parabènes.

3. Composition pharmaceutique selon la revendication 1 ou 2, dans laquelle l'agent d'isotonie est choisi dans un groupe contenant le mannitol, le sorbitol, le lactose, le D-glucose, le tréhalose, le chlorure de sodium, le glycérol.

4. Composition pharmaceutique selon l'une quelconque des revendications 1 à 3, dans laquelle les adjuvants sont choisis dans un groupe contenant des substances tampon comme par exemple Tris, phosphate, citrate, acétate, glycylglycine ou d'autres substances telles que des acides, des solutions alcalines, des sels.

5. Composition pharmaceutique selon l'une quelconque des revendications précédentes, dans laquelle la Gly(A21), Arg(B31), Arg(B32)-insuline humaine est présente à une concentration de 60 - 6 000 nmoles/ml.

6. Composition pharmaceutique selon la revendication 5, dans laquelle la Gly(A21), Arg(B31), Arg(B32)-insuline humaine est présente à une concentration de 240 - 3 000 nmoles/ml.

7. Composition pharmaceutique selon la revendication 6, dans laquelle le tensioactif est présent à une concentration de 5 - 120 μg/ml.

8. Composition pharmaceutique selon la revendication 7, dans laquelle le tensioactif est présent à une concentration de 20 - 75 μg/ml.

9. Composition pharmaceutique selon une ou plusieurs des revendications 4 à 8, dans laquelle le glycérol et/ou le mannitol est/sont présent(s) à une concentration de 100-250 mM.

10. Composition pharmaceutique selon la revendication 9, dans laquelle le NaCl est présent à une concentration de jusqu'à 150 mM.

11. Composition pharmaceutique selon l'une quelconque des revendications précédentes, dans laquelle ladite une substance tampon est présente à une concentration de 5 - 250 mM.

12. Composition pharmaceutique selon l'une quelconque des revendications précédentes, dans laquelle le tensioactif est le Tween® 20.

13. Composition pharmaceutique selon l'une quelconque des revendications précédentes, dans laquelle le tensioactif est le Tween® 80.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0214826 A
- EP 0375437 A
- EP 0678522 A
- EP 0124826 A
- EP 0419504 A
- WO 9200321 A
- EP 0368187 A
- WO 0143762 A
- WO 0023098 A
- EP 0202625 W
- US 4994439 A
- FR 2456522
- DE 4140186 A1

**In der Beschreibung aufgeführte Nicht-Patentliteratur**

- *N. Engl. J. Med.,* 1993, vol. 329, 977-986
- **BRANGE et al.** *J. Ph.Sci,* 1997, vol. 86, 517-525
- **SLUZKY et al.** *Proc.Natl.Acad.Sci.,* 1991, vol. 88, 9377-9381